# FASCICULE DE BREVET EUROPEEN

(11) **EP 2 752 613 B1**
(45) Date de publication et mention de la délivrance du brevet: **14.09.2016**
(21) Numéro de dépôt: 13198572.3
(22) Date de dépôt: 19.12.2013
(51) Int. Cl.: F21L 14/04, A61N 5/06, E04H 12/18, F16M 11/20, F21V 17/00, F21V 21/06, F21V 21/26, F21V 21/28, F16M 11/24, F16M 11/38, F16M 11/42, F16M 11/28, F21W 131/205, F21Y 115/10

(54) **Dispositif d'éclairage médical**
Medizinische Beleuchtungsvorrichtung
Medical lighting device

(30) Priorité: 04.01.2013 FR 1350072
(43) Date de publication de la demande: 09.07.2014
(73) Titulaire: Steris, 33185 Le Haillan (FR)
(72) Inventeur: L'Hégarat, Jean-Marie, 33160 Saint Aubin de Médoc (FR); Besnard, Mathieu, 33160 Saint Médard en Jalles (FR)
(74) Mandataire: Robert, Mathias

(56) Documents cités:
- EP-A1- 0 106 395
- WO-A1-03/058115
- CN-U- 201 944 559
- DE-A1- 2 849 526
- DE-A1- 19 904 473
- FR-A1- 2 529 968
- NL-C2- 1 022 407
- US-A- 2 069 950
- US-A- 3 310 673
- US-A- 4 866 284
- US-A- 4 867 416
- US-A- 6 005 254
- US-B2- 7 174 982
- US-B2- 8 297 780

## Description

L'invention concerne un dispositif d'éclairage médical, notamment pour bloc opératoire.

Un tel dispositif d'éclairage comporte classiquement un bras articulé dont une extrémité est fixe et dont l'autre extrémité est mobile et supporte des moyens d'éclairage. L'extrémité fixe est par exemple fixée à un mur, à un plafond ou encore à un bras de distribution d'un bloc opératoire. Le bras articulé est formé de plusieurs éléments reliés les uns aux autres par des articulations permettant de déplacer et d'orienter manuellement les moyens d'éclairage.

A des fins de démonstration chez des clients, il a été envisagé de développer des dispositifs d'éclairage pouvant être transportés facilement puis installés rapidement sur le site de démonstration.

De tels dispositifs transportables peuvent également être utilisés pour d'autres applications, par exemple en vue d'équiper des postes médicaux avancés déployés lors de conflits ou lors d'évènements majeurs ou de crise.

L'invention vise à répondre à cette problématique grâce à un dispositif d'éclairage pouvant être transporté aisément, tout en étant d'installation facile et rapide.

A cet effet, l'invention propose un dispositif d'éclairage médical comportant un socle destiné à être posé au sol, un mât dont la première extrémité est reliée au socle et dont la seconde extrémité supporte un bras, ledit bras comportant une première extrémité articulée sur le mât et une seconde extrémité supportant des moyens d'éclairage, le mât étant mobile entre une position rétractée et une position déployée.

Le dispositif d'éclairage peut ainsi être transporté facilement, en position rétractée du mât, puis déployé sur le site de démonstration par exemple.

De préférence, le mât est télescopique et comporte plusieurs segments pouvant coulisser les uns par rapport aux autres.

Dans ce cas, le déploiement du bras télescopique est obtenu par actionnement d'une pompe, par exemple actionnée manuellement ou à l'aide d'une pédale.

Un mât télescopique actionné de façon pneumatique est notamment connu du document US 2012/0079778, pour d'autres applications.

Selon une caractéristique de l'invention, le mât comporte des moyens de verrouillage permettant d'interdire le coulissement des différents éléments du mât les uns par rapport aux autres.

Ceci permet notamment de sécuriser le dispositif d'éclairage une fois déployé.

En outre, le socle peut comporter des roues de transport, afin de faciliter le déplacement du dispositif d'éclairage.

Selon une forme de réalisation de l'invention, la première extrémité du bras est reliée à la seconde extrémité du mât par l'intermédiaire d'une articulation comportant une liaison pivot d'axe parallèle au mât et décalé de l'axe du mât et/ou une liaison pivot autour d'un axe perpendiculaire au mât.

Une telle architecture permet de replier facilement et de manière compacte le bras le long du mât. Ainsi, en position repliée ou rétractée, l'encombrement du dispositif d'éclairage est relativement réduit, ce qui facilite son transport et son installation sur site.

Dans ce cas, le dispositif d'éclairage peut comporter des butées permettant de limiter la rotation du bras articulé par rapport au mât, selon l'axe parallèle au mât, entre deux positions limites.

Les butées sont positionnées de façon à éviter un basculement du dispositif d'éclairage lors du déplacement des moyens d'éclairage et lorsque le mât est déployé.

Selon une autre caractéristique de l'invention, la première extrémité du mât est reliée au socle par l'intermédiaire d'une articulation comportant une liaison pivot d'axe perpendiculaire à l'axe du mât.

Une telle caractéristique permet encore d'améliorer la compacité du dispositif d'éclairage en position repliée ou rétractée.

En outre, la première extrémité du mât est située à proximité d'un bord du socle, au moins un contrepoids étant monté sur le socle, à l'opposé de la première extrémité du mât.

De cette manière, il est possible de libérer un large espace de travail pour un opérateur ou un chirurgien, placé à proximité du mât et sous le bras et les moyens d'éclairage.

Selon l'invention, une platine relie la seconde extrémité du mât et la première extrémité du bras.

Ladite platine comporte une première partie reliée à la seconde extrémité du mât et une seconde partie reliée à la première extrémité du bras, les deux parties étant pivotantes l'une par rapport à l'autre autour d'un axe perpendiculaire au mât.

Une telle caractéristique permet encore d'améliorer la compacité du dispositif d'éclairage lorsque celui-ci est en position repliée ou rétractée.

En outre, au moins l'une des articulations comporte des moyens de verrouillage permettant d'interdire tout mouvement relatif entre les éléments articulés correspondants, de manière à pouvoir maintenir le dispositif d'éclairage en position déployée par exemple.

L'invention sera mieux comprise et d'autres détails, avantages et caractéristiques de l'invention apparaîtront à la lecture de la description suivante faite à titre d'exemple non limitatif, en référence aux dessins annexés dans lesquels :
- la figure 1 est une vue en perspective d'un dispositif d'éclairage n'appartenant pas à l'invention, en position rétractée du mât ;
- la figure 2 est une vue correspondant à la figure 1, dans laquelle le mât est en position déployée,
- la figure 3 est une vue de côté du dispositif d'éclairage, en position déployée à côté d'un opérateur et d'une table d'opération sur laquelle est allongé un patient,
- la figure 4 est une vue en perspective de ce dispositif d'éclairage en position déployée,
- la figure 5 est une vue de détail de la figure 4, illustrant le montage des butées sur la platine,
- la figure 6 est une vue de détail, en perspective, illustrant une pompe à main servant au déploiement du mât télescopique,
- la figure 7 est une vue en coupe de l'extrémité supérieure du mât, illustrant notamment la structure d'un connecteur électrique tournant,
- les figures 8 à 11 sont des vues en perspective illustrant différentes étapes de repliement et de conditionnement du dispositif d'éclairage, en vue de son transport,
- la figure 12 est une vue de côté d'un deuxième dispositif d'éclairage n'appartenant pas à l'invention,
- les figures 13 à 24 illustrent un dispositif d'éclairage selon une forme de réalisation selon l'invention, à savoir :

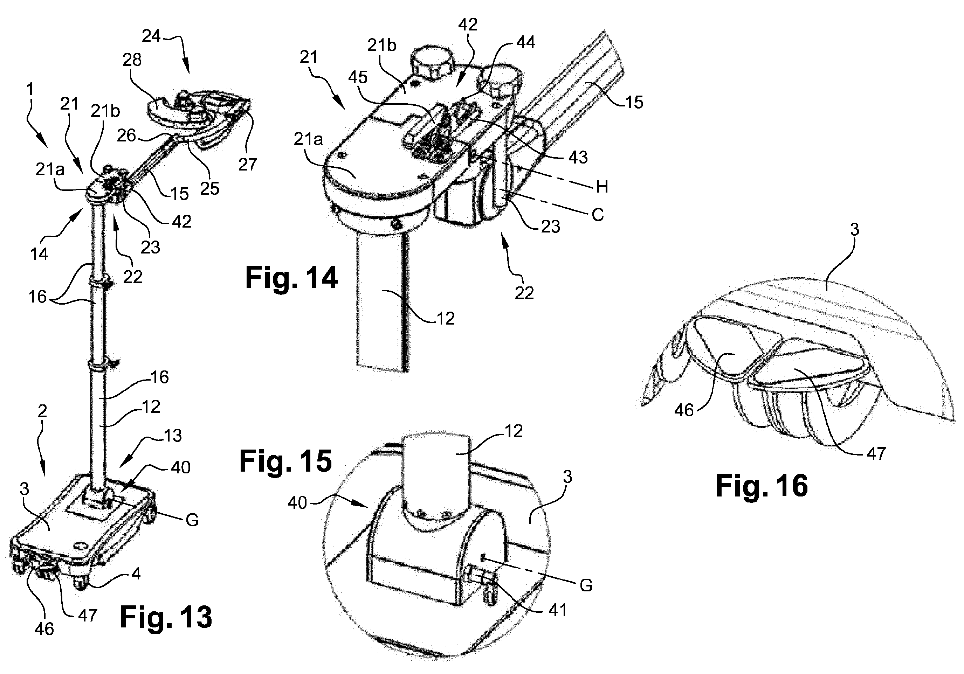

- la figure 13 est une vue en perspective du dispositif d'éclairage,
- les figures 14, 15 et 16 sont des vues de détail de certaines parties du dispositif d'éclairage,

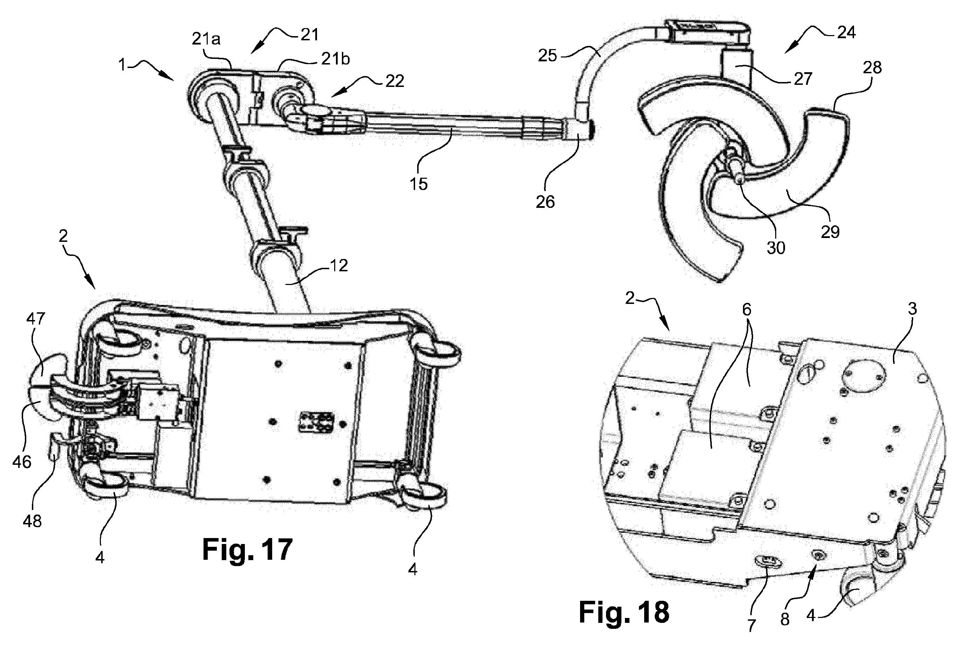

- la figure 17 est une vue en perspective, de dessous, du dispositif d'éclairage,
- la figure 18 est une vue de détail d'une partie du socle du dispositif d'éclairage,

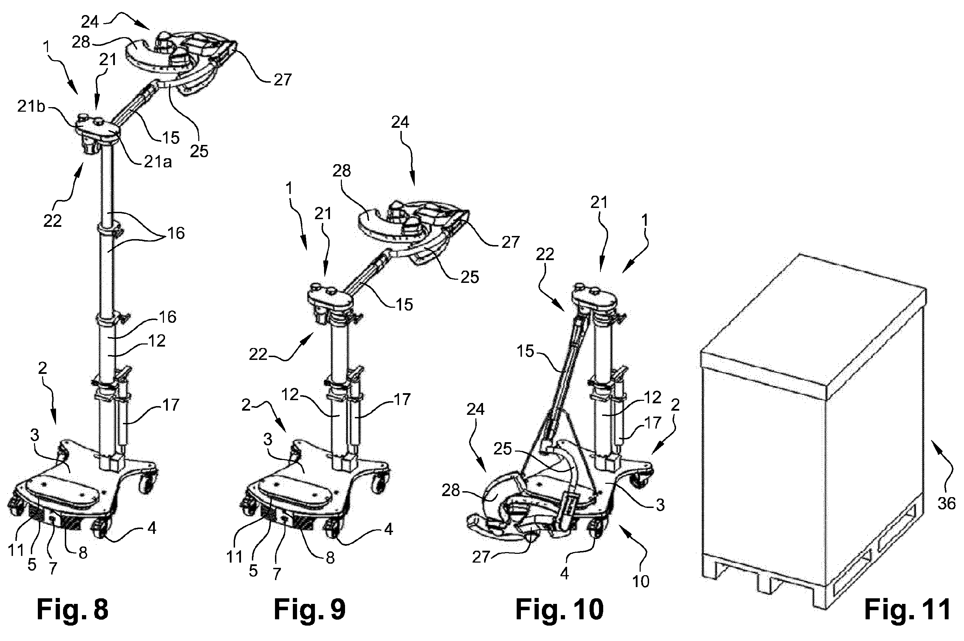

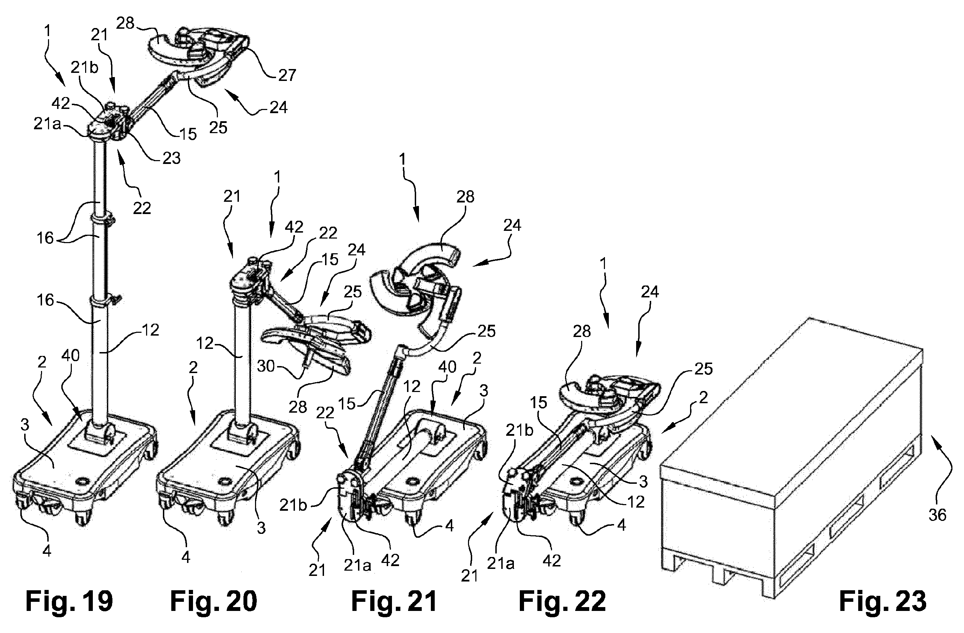

- les figures 19 à 23 sont des vues correspondant respectivement aux figures 8 à 11, du dispositif d'éclairage selon la forme de réalisation,

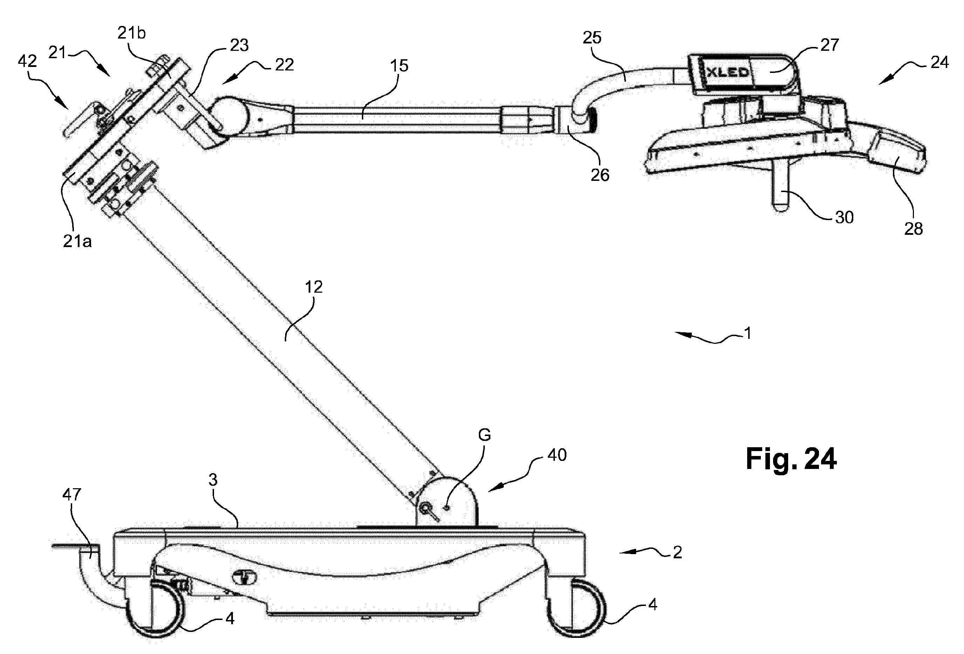

- la figure 24 est une vue de côté du dispositif d'éclairage dans une position intermédiaire,

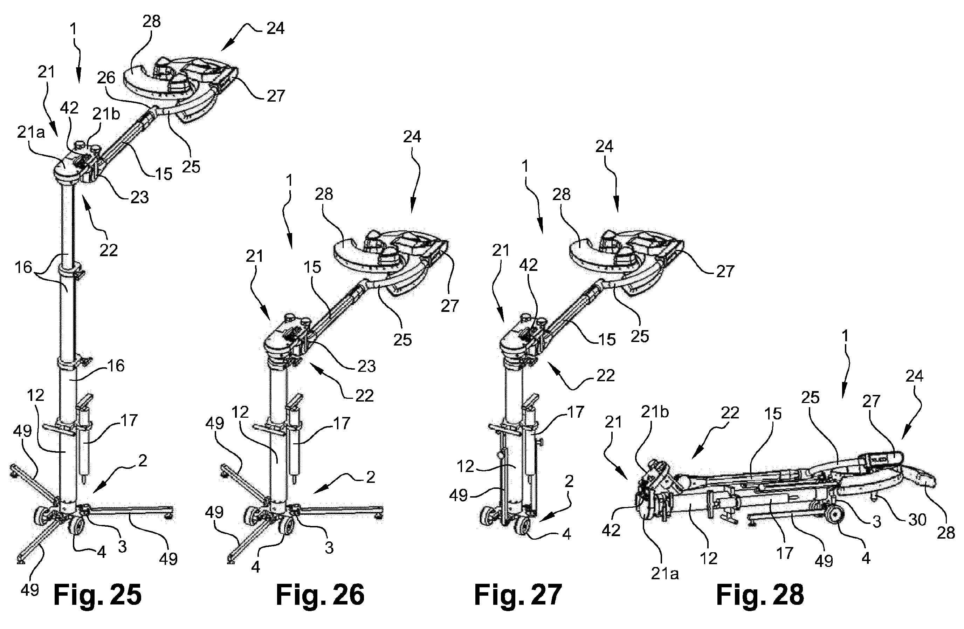

- les figures 25 à 28 sont des vues correspondant respectivement aux figures 8 à 11, du dispositif d'éclairage selon une autre forme de réalisation,

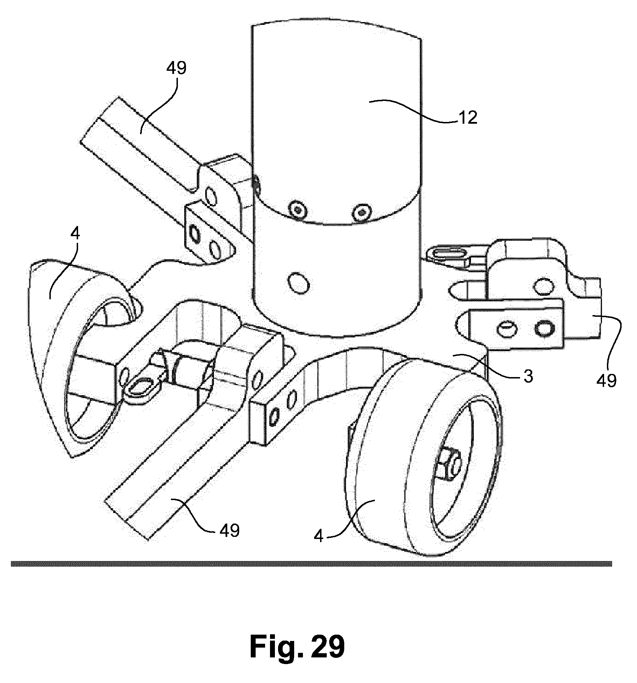

- la figure 29 est une vue de détail d'une partie du dispositif d'éclairage des figures 25 à 28.

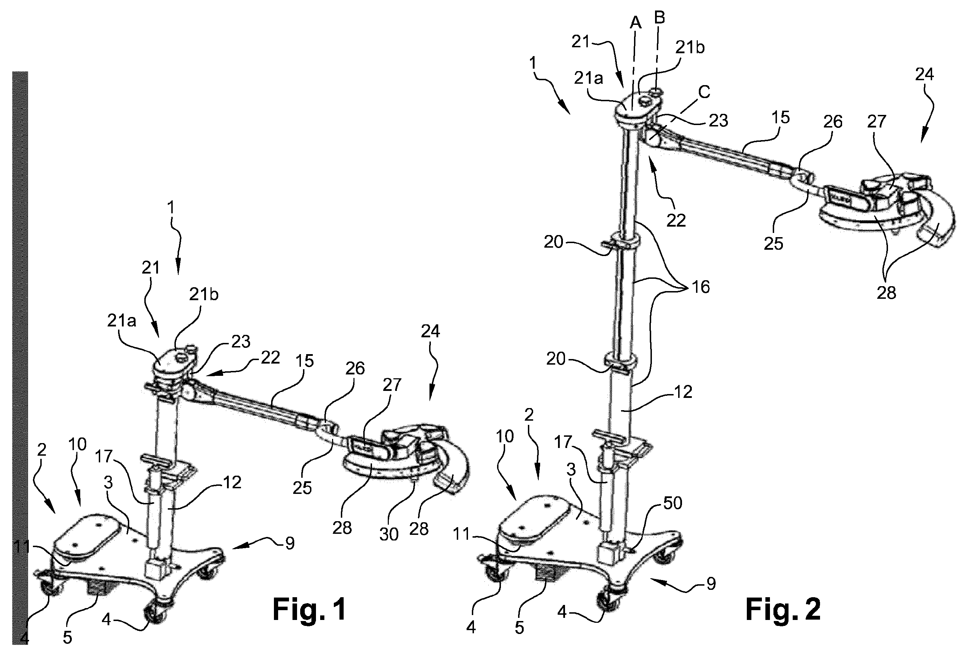

Un dispositif d'éclairage médical 1 est illustré aux figures 1 à 11 et comporte un socle 2 destiné à être posé au sol et comprenant un support 3 sensiblement plan et horizontal sur lequel sont montées quatre roulettes 4 écartées les unes des autres. Le socle 2 comporte des moyens d'alimentation électrique 5 pouvant comprendre une batterie 6 (figure 18) et/ou des moyens de raccordement à un réseau électrique, tels qu'une fiche standard 7 (prise secteur) et d'une prise de type équipotentielle 8 (masse), comme représenté aux figures 8 et 9. Le support 3 comporte une extrémité dite avant 9, située à droite sur la figure 1, et une extrémité arrière 10. La partie arrière du support 3 comporte un contrepoids 11 qui peut être formé, au moins en partie, par les batteries par exemple. Les roulettes 4 situées au niveau de l'extrémité arrière 10 du support 3 comportent des moyens de verrouillage empêchant le déplacement du socle 2.

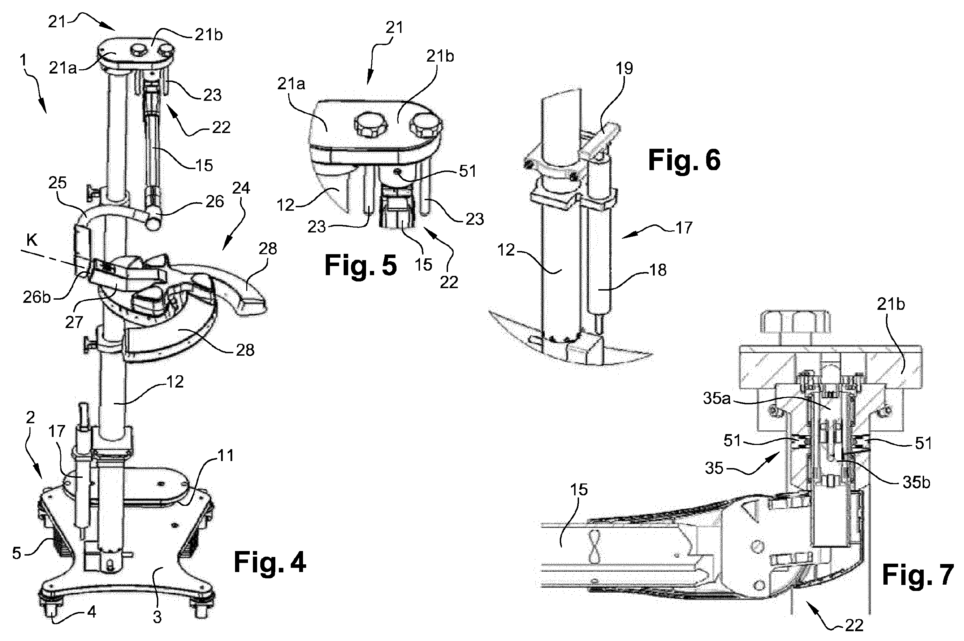

Un mât 12 est monté sur la partie avant du support 3, en un point décalé latéralement par rapport au plan médian longitudinal du support 3, comme cela est mieux visible à la figure 4.

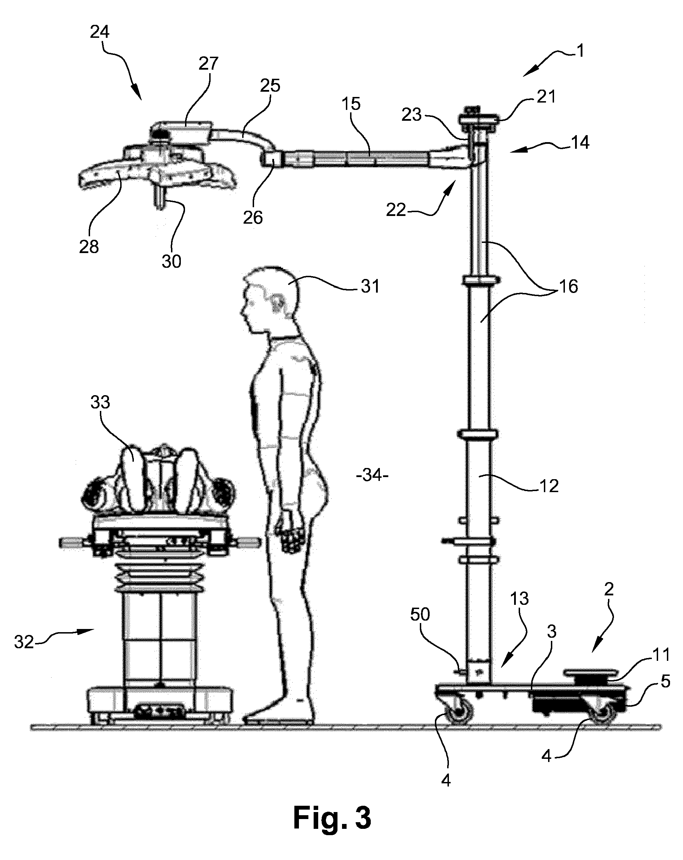

Le mât 12 comporte une première extrémité 13 fixée au support 3 et une seconde extrémité 14 supportant un bras 15 (voir notamment figure 3). Le mât 12 est du type télescopique et comporte plusieurs segments 16 pouvant coulisser les uns par rapport aux autres. Le mât 12 comporte un dispositif pneumatique permettant de le déployer lorsque l'on augmente la pression à l'aide d'une pompe 17, et de le rétracter progressivement par abaissement de la pression (purge).

Un tel bras télescopique est par exemple connu du document US 2012/0079778.

Dans le cas de l'invention toutefois, la pompe 17 est une pompe actionnée manuellement, comportant un corps cylindrique 18 fixé sur le mât 12, et une poignée 19 reliée à un piston coulissant par rapport au corps 18 (figure 6). Lorsqu'un opérateur actionne la poignée 19, il augmente la pression à l'intérieur du mât 12 et permet ainsi son déploiement. La position déployée du mât 12 est illustrée à la figure 2 notamment. Un bouton 50 (visible sur les figures 2 et 3) permet de réaliser une purge de l'air et rétracter ainsi progressivement le mât 12. La position rétractée ou abaissée du mât 12 est illustrée à la figure 1 notamment.

Le mât comporte également des moyens de verrouillage 20 permettant d'empêcher le déplacement des différents secteurs les uns par rapport aux autres (figure 2).

En variante, le bras 12 pourrait être actionné à l'aide d'un dispositif hydraulique, électromécanique ou uniquement de façon mécanique.

Une platine 21 s'étendant perpendiculairement au mât 12 est fixée sur une seconde extrémité 14 du mât 12. Plus particulièrement, la platine 21 comporte une première zone 21 a de fixation à la seconde extrémité 14 du mât 12 et une seconde zone 21 b sur laquelle est articulé le bras 15. Cette articulation 22 est formée par une liaison pivot selon un axe B parallèle à l'axe A du mât 12 et décalé de ce dernier, et par une liaison pivot selon un axe C perpendiculaire à l'axe A du mât et à la direction longitudinale du support 3.

Comme cela est mieux visible à la figure 5, deux tiges de butée 23 sont fixées de manière amovible sur la seconde zone 21 b de la platine 21 et font saillie de part et d'autre du bras 15. De cette manière, le pivotement du bras 15 selon l'axe B est limité par appui sur les butées 23. La plage angulaire autorisée par les butées 23 est par exemple comprise entre - 15° et + 15°. On notera par ailleurs que la plage angulaire autorisé par le pivot selon l'axe C est comprise entre + 30° et - 45°. Ces plages angulaires sont suffisantes pour les applications visées.

La seconde extrémité du bras 15 supporte des moyens d'éclairage 24 similaires à ceux décrits dans la demande de brevet FR 2 975 460, au nom de la Demanderesse. Ces moyens d'éclairage 24 comportent notamment un bras coudé 25 dont une extrémité est articulée à l'aide d'une liaison pivot 26 autour de l'axe du bras 15, et dont l'autre extrémité est articulée sur un boîtier 27 supportant des modules 28 de forme hélicoïdale. L'articulation entre le bras 25 et le boîtier 27 est référencée 26b et est formée d'une liaison pivot autour d'un axe K perpendiculaire à l'axe du bras 15 (figure 4). Chaque module 28 comporte notamment des diodes électroluminescentes associées à des collimateurs et à des éléments optiques tels que des plaques de protection 29 (figure 17) et/ou des lentilles fixes ou mobiles. Les diodes électroluminescentes sont situées sur la surface externe d'une sphère, lesdites diodes éclairant ainsi une zone autour du centre de la sphère. Une poignée 30 faisant saillie du boîtier 27 permet à un opérateur de positionner et d'orienter correctement les moyens d'éclairage 24, en agissant sur les articulations 22, 26 et 26b précitées des bras 15 et 25.

Le bras 15 peut être bloqué dans la position voulue à l'aide de moyens connus, notamment à l'aide de ressorts ou de vérins à gaz capables de maintenir le bras 15 et les moyens d'éclairage 24 en position. Des moyens de freinage 51 permettant de maintenir ou de freiner le bras 15 en position autour de l'axe B sont visibles aux figures 5 et 7.

La figure 3 illustre le dispositif d'éclairage 1 dans une position de travail, par exemple à l'intérieur d'un bloc opératoire. Le socle 2 et le mât 12 sont alors positionnés derrière un opérateur 31, par exemple derrière un chirurgien, et les moyens d'éclairage 24 sont positionnés et orientés de manière à éclairer un patient 33 allongé sur une table d'opération 32 ou sur une table d'un cabinet dentaire par exemple. Dans cette position, le mât 12 est déployé de façon à ce que le bras 15 s'étende au-dessus de l'opérateur 31. D'autres applications médicales sont évidemment envisageables.

On remarque que, dans cette position, un espace de travail libre 34 est préservé entre la table 32, d'une part, et le socle 3 ou le mât 12, d'autre part, de façon à ne pas entraver les mouvements de l'opérateur 31. Ceci est notamment dû au fait que le mât 12 est fixé au niveau d'une extrémité du socle 3, le reste du socle 3 étant situé à l'opposé du bras 15 et des moyens d'éclairage 24 par rapport au mât 12, en position déployée du dispositif d'éclairage. Dans ce cas toutefois, il est nécessaire de placer un contrepoids 11 correctement dimensionné à l'opposé du bras 15 et des moyens d'éclairage 24 par rapport au mât 12, de façon à éviter le basculement du dispositif d'éclairage 1.

La présence des butées 23 permettant de limiter la rotation du bras 15 autour de l'axe B sert également à éviter un basculement du dispositif d'éclairage 1.

Les moyens d'alimentation électrique 5 du socle 2 sont reliés aux différents modules d'éclairage 28 par l'intermédiaire de câbles électriques traversant notamment le mât 12, le bras 15 et le bras coudé 25, et par l'intermédiaire de connecteurs électriques tournants 35, tels que celui représenté à la figure 7, assurant la continuité électrique au niveau de certaines articulations. La figure 7 représente un connecteur tournant 35 comportant une première partie 35a montée sur la platine 21 et une seconde partie 35b, mobile par rapport à la première partie 35a, montée au niveau de la première extrémité du bras 15. Les deux parties 35a, 35b du connecteur 35 assurent la connexion électrique quelles que soient leurs positions angulaires respectives.

Les câbles et les connecteurs précités peuvent également permettre la transmission de signaux, tels que des signaux de commande, entre les différents éléments du dispositif d'éclairage 1.

Après utilisation, le dispositif d'éclairage 1 qui est dans la position représentée à la figure 8 doit être replié en vue de son transport. Pour cela, le mât 12 est rétracté ou abaissé (figure 9), et l'une au moins des butées amovibles 23 est retirée de la platine 21, de façon à permettre la rotation du bras 15 autour de l'axe B. Par rotation du bras 15 selon les axes B et C, les moyens d'éclairage sont positionnés à proximité de l'extrémité 10 du support 3, de façon à limiter l'encombrement de l'ensemble du dispositif d'éclairage 1 (figure 10). Ce dernier peut alors être logé dans une caisse 36 de taille relativement réduite, en vue de son transport (figure 11).

Dans une variante non représentée, il est possible d'utiliser un bras 15 articulé sur la platine 21 du mât télescopique 12 uniquement à l'aide d'un pivot selon l'axe C. Dans ce cas il convient de pouvoir faire pivoter la platine 21 par rapport au socle 2. Ceci peut être réalisé par une liaison pivot entre la platine 21 et la seconde extrémité 14 du mât 12, par une liaison pivot entre la première extrémité 13 du mât 12 et le support 3, ou encore par des liaisons de type pivots glissants entre au moins deux segments 16 du mât télescopique 12.

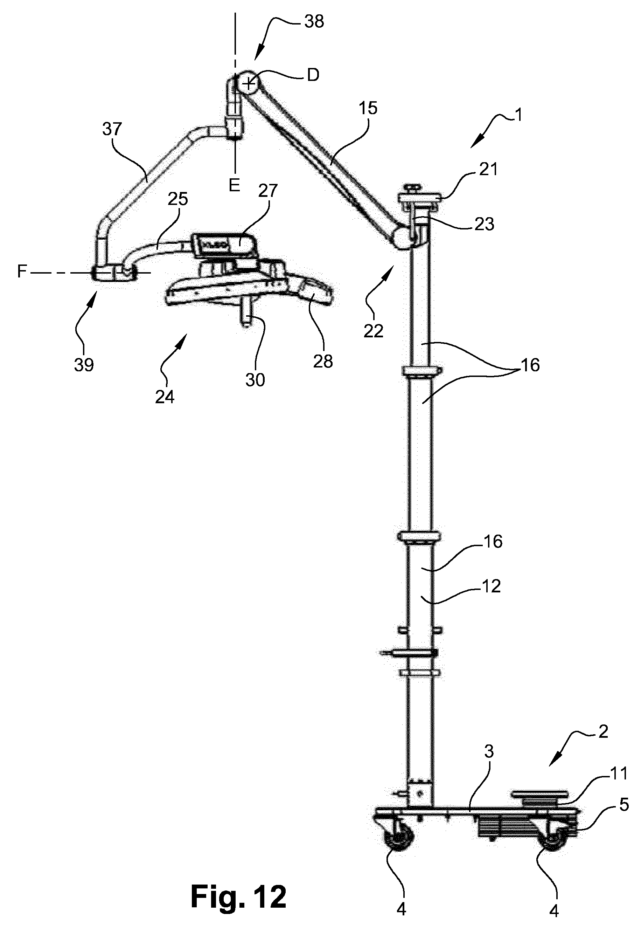

La figure 12 illustre une deuxième dispositif d'éclairage dans laquelle les moyens d'éclairage 24 comportent un bras intermédiaire 37 reliant la seconde extrémité du bras 15 au bras coudé 25. Le bras intermédiaire 37 est articulé sur le bras 15 et sur le bras coudé 25, par l'intermédiaire d'articulations respectivement référencées 38 et 39. L'articulation 38 est composée de deux liaisons pivots perpendiculaires, d'axes respectifs D et E. L'axe D est parallèle à l'axe C et l'axe E est parallèle aux axes A et B. L'articulation 39 est formée d'une liaison pivot d'axe F, perpendiculaire aux axes D et E. Une telle variante permet une plus grande maniabilité et facilite le positionnement et l'orientation des moyens d'éclairage 24 par rapport à la zone à éclairer.

Les figures 13 à 24 illustrent une forme de réalisation du dispositif d'éclairage 1, dans laquelle la première extrémité 13 du mât 12 est montée dans la zone avant du support 3 et au niveau du plan médian longitudinal du support 3. De plus, la première extrémité 13 du mât 12 est articulée sur le support 3 au moyen d'une articulation 40 formée par une liaison pivot d'axe G perpendiculaire à l'axe A du mât 12 et au plan médian longitudinal du support 3. Le mât 12 est ainsi mobile entre une position de travail ou position déployée dans laquelle il est vertical ou perpendiculaire au support 3 (figure 13), et une position de transport ou position rabattue dans laquelle il est rapproché du support 3 (figures 21 et 22). Cette articulation 40 autorise un débattement angulaire de 90° entre le mât 12 et le support 3. Elle comporte des moyens de verrouillage permettant d'immobiliser le mât 12 dans sa position de travail et/ou dans sa position de transport. Ces moyens de verrouillage se présentent par exemple sous la forme d'un doigt d'indexation bistable 41, destiné à être inséré dans des trous ménagés dans des parties de l'articulation 40 solidaires respectivement du mât 12 et du support 3 (figure 15).

En outre, la platine 21 comporte une première partie 21 a fixée sur la seconde extrémité 14 du mât 12 et une seconde partie 21 b supportant le bras 15, les deux parties 21 a, 21 b étant articulées autour d'un axe H parallèle à l'axe G d'articulation du mât 12 sur le support 3 (figure 14). La seconde partie 21 b est ainsi mobile par rapport à la première partie 21 a, entre une position déployée ou position de travail dans laquelle les deux parties s'étendent sensiblement dans le même plan (figure 14) et une position rabattue ou position de transport dans laquelle la seconde partie 21 b forme un angle avec la première partie 21 a (figure 22). Cette articulation autorise un débattement angulaire d'environ 45° entre les deux parties 21 a, 21 b. Des moyens de verrouillage 42 permettent d'immobiliser les deux parties 21 a, 21 b dans la position de travail. Ces moyens de verrouillage 42 (mieux visibles à la figure 14) sont par exemple de type sauterelle et comportent alors un fil 43 en forme de U, rattaché à l'une des parties 21 a, venant s'insérer dans au moins un crochet 44 de l'autre partie 21 b. Le fil 43 est amené ou dégagé du crochet 44 par actionnement d'une poignée 45.

Dans cette forme de réalisation, la première extrémité du bras 15 est articulée sur la seconde partie 21b de la platine 21, par l'intermédiaire de l'articulation 22 comportant alors une unique liaison pivot d'axe C parallèle à l'axe H d'articulation des deux parties 21 a, 21 b de la platine 21 et parallèle à l'axe G d'articulation du mât 12 sur le support 3.

Dans cette forme de réalisation également, l'actionnement pneumatique du mât 12 n'est pas réalisé à l'aide d'une pompe à main, mais à l'aide d'une pompe actionnée par des pédales 46, 47 et dissimulée dans le socle 2. Plus particulièrement, une première pédale 46 permet, par actionnements successifs, d'augmenter la pression du circuit pneumatique afin de déployer le mât télescopique 12. Une autre pédale 47 permet, au contraire, de réduire la pression du circuit pneumatique afin d'abaisser le bras 12. Une autre pédale 48 (figure 17) peut servir à verrouiller ou déverrouiller les roulettes 4 afin d'immobiliser le socle 2 par rapport au sol.

Après utilisation, le dispositif d'éclairage 1 qui est dans la position représentée à la figure 19 doit être replié en vue de son transport. Pour cela, le mât 12 est rétracté ou abaissé (figure 20), puis le mât 12 est pivoté par rapport au socle 2 vers sa position repliée (figure 21). Enfin, la seconde partie 21 b de la platine 21 peut être repliée et le bras 15 peut être pivoté par rapport à la seconde partie 21 b de la platine 21, de manière à ce que le bras 15, le mât 12 et le support 3 soient sensiblement parallèles (figure 22). L'encombrement de l'ensemble du dispositif d'éclairage 1 est ainsi considérablement réduit. Ce dernier peut alors être logé dans une caisse 36 de taille très réduite, en vue de son transport (figure 23). Le volume de la caisse 36 illustrée à la figure 23 est au moins inférieur de 30 % au volume de la caisse 36 illustrée à la figure 11.

On notera que, dans cette forme de réalisation, il n'est pas nécessaire de retirer les butées amovibles 23 de la platine 21.

Les articulations 22 et 40 peuvent être mues par un dispositif pneumatique, hydraulique, électromécanique ou uniquement mécanique.

Comme cela est représenté à la figure 24, les moyens de verrouillage 41 équipant l'articulation 40 peuvent également permettre d'immobiliser le mât 12 dans une position dans laquelle il forme un angle de 45° avec le support 3. Dans cette position également, les moyens de verrouillage 42 maintiennent les deux parties 21 a, 21 b de la platine 21 dans le même plan.

Les figures 25 à 28 illustrent une autre forme de réalisation de l'invention, se distinguant de celle des figures 13 à 23 en ce que le socle 2 comporte un support 3 de taille réduite, fixé à la première extrémité 13 du mât 12 et portant deux roulettes 4. Trois pieds 49 décalés angulairement les uns des autres sont articulés sur le support 3, entre une position repliée dans laquelle ils s'étendent le long du mât 12, et une position déployée dans laquelle ils sont destinés à prendre appui sur le sol. Des moyens de verrouillage se présentant sous la forme d'un doigt d'indexation 52 (figure 29) permettent de maintenir les pieds 49 en position déployée, de manière à ce que les pieds 49 forment un trépied capable de soutenir le mât 12 en position verticale, quelle que soit la position des moyens d'éclairage 24 et du bras articulé 15.

Après utilisation, le dispositif d'éclairage 1 qui est dans la position représentée à la figure 25 doit être replié en vue de son transport. Pour cela, le mât 12 est rétracté ou abaissé (figure 26), puis les pieds 49 sont repliés le long du mât 12 (figure 27). Enfin, la seconde partie 21 b de la platine 21 peut être repliée et le bras 15 peut être pivoté par rapport à la seconde partie 21b de la platine 21, de manière à ce que le bras 15, le mât 12 et le support 3 soient sensiblement parallèles (figure 28). L'encombrement de l'ensemble du dispositif d'éclairage 4 est alors considérablement réduit. On notera que, dans ce cas, il n'est pas nécessaire que le mât 12 soit articulé sur le support 3.

## Revendications

1. Dispositif d'éclairage médical (1) comportant un socle (2) destiné à être posé au sol, un mât (12) dont la première extrémité (13) est reliée au socle (2) et dont la seconde extrémité (14) supporte un bras (15), ledit bras (15) comportant une première extrémité articulée (22) sur le mât (12) et une seconde extrémité supportant des moyens d'éclairage (24), le mât (12) étant mobile entre une position rétractée et une position déployée, **caractérisée en ce qu'**une platine (21) relie la seconde extrémité (14) du mât (12) et la première extrémité du bras (15), ladite platine (21) comportant une première partie (21a) reliée à la seconde extrémité (14) du mât (12) et une seconde partie (21b) reliée à la première extrémité (13) du mât (12), les deux parties (21a, 21b) étant pivotantes l'une par rapport à l'autre autour d'un axe (H) perpendiculaire au mât (12).

2. Dispositif d'éclairage médical (1) selon la revendication 1, **caractérisé en ce que** le mât (12) est télescopique et comporte plusieurs segments pouvant (16) coulisser les uns par rapport aux autres.

3. Dispositif d'éclairage médical (1) selon la revendication 2, **caractérisé en ce que** le déploiement du bras télescopique (12) est obtenu par actionnement d'une pompe, par exemple actionnée manuellement ou à l'aide d'une pédale (46, 47).

4. Dispositif d'éclairage médical (1) selon la revendication 2 ou 3, **caractérisé en ce que** le mât (12) comporte des moyens de verrouillage (20) permettant d'interdire le coulissement des différents éléments (16) du mât (12) les uns par rapport aux autres.

5. Dispositif d'éclairage médical (1) selon l'une des revendications 1 à 4, **caractérisé en ce que** le socle (2) comporte des roues (4) de transport.

6. Dispositif d'éclairage médical selon l'une des revendications 1 à 5, **caractérisé en ce que** la première extrémité (13) du bras (12) est reliée à la seconde extrémité du mât par l'intermédiaire d'une articulation (22) comportant une liaison pivot d'axe (B) parallèle au mât (12) et décalé de l'axe (A) du mât (12) et/ou une liaison pivot autour d'un axe (C) perpendiculaire au mât (12).

7. Dispositif d'éclairage médical (1) selon la revendication 6, **caractérisé en ce qu'**il comporte des butées (23) permettant de limiter la rotation du bras articulé (15) par rapport au mât (12), selon l'axe (B) parallèle au mât (12), entre deux positions limites.

8. Dispositif d'éclairage médical (1) selon l'une des revendications 1 à 7, **caractérisé en ce que** la première extrémité (13) du mât (12) est reliée au socle (2) par l'intermédiaire d'une articulation (40) comportant une liaison pivot d'axe (G) perpendiculaire à l'axe (A) du mât (12).

9. Dispositif d'éclairage médical (1) selon l'une des revendications 1 à 8, **caractérisé en ce que** la première extrémité (13) du mât (12) est située à proximité d'un bord (13) du socle (2), au moins un contrepoids (11) étant monté sur le socle (2), à l'opposé de la première extrémité (13) du mât (12).

10. Dispositif d'éclairage médical (1) selon l'une des revendications 1 à 9, **caractérisé en ce qu'**au moins l'une des articulations comporte des moyens de verrouillage (41, 45) permettant d'interdire tout mouvement relatif entre les éléments articulés correspondants.

## Patentansprüche

1. Medizinische Beleuchtungsvorrichtung (1) mit einem Sockel (2), der dazu bestimmt ist, auf den Boden gestellt zu werden, einem Masten (12), dessen erstes Ende (13) mit dem Sockel (2) verbunden ist, und dessen zweites Ende (14) einen Arm (15) stützt, wobei besagter Arm (15) ein erstes angelenktes Ende (22) auf dem Mast (12) und ein zweites, die Beleuchtungsmittel (24) stützendes Ende umfasst, wobei der Mast (12) zwischen einer eingefahrenen und einer ausgefahrenen Position bewegt werden kann, **dadurch gekennzeichnet, dass** eine Platte (21) das zweite Ende (14) des Mastes (12) mit dem ersten Ende des Arms (15) verbindet, wobei besagte Platte (21) einen ersten Teil (21a) umfasst, der mit dem zweiten Ende (14) des Mastes (12) verbunden ist, und einen zweiten Teil (21b), der mit dem ersten Ende (13) des Mastes (12) verbunden ist, wobei die beiden Teile (21a, 21b) gegeneinander um eine senkrecht zum Mast (12) verlaufende Achse (H) gedreht werden können.

2. Medizinische Beleuchtungsvorrichtung (1) nach Anspruch 1, **dadurch gekennzeichnet, dass** der Mast (12) ausfahrbar ist und mehrere Abschnitte (16) enthält, die gegeneinander verschoben werden können.

3. Medizinische Beleuchtungsvorrichtung (1) nach Anspruch 2, **dadurch gekennzeichnet, dass** das Ausfahren des Teleskoparms (12) durch Betätigen einer Pumpe erzielt wird, wobei diese zum Beispiel manuell oder mit Hilfe eines Pedals (46, 47) betätigt werden kann.

4. Medizinische Beleuchtungsvorrichtung (1) nach Anspruch 2 oder 3, **dadurch gekennzeichnet, dass** der Mast (12) Verriegelungsmittel (20) aufweist, die verhindern können, dass die Elemente (16) des Mastes (12) gegeneinander verschoben werden.

5. Medizinische Beleuchtungsvorrichtung (1) nach einem der Ansprüche 1 bis 4, **dadurch gekennzeichnet, dass** der Sockel (2) Transporträder (4) umfasst.

6. Medizinische Beleuchtungsvorrichtung (1) nach einem der Ansprüche 1 bis 5, **dadurch gekennzeichnet, dass** das erste Ende (13) des Arms (12) mit dem zweiten Ende des Mastes über ein Gelenk (22) verbunden ist, das eine parallel zum Mast (12) verlaufende Drehzapfenverbindung von Achse (B) aufweist und versetzt zur Achse (A) des Mastes (12) angeordnet ist, und/oder eine Drehzapfenverbindung um eine Achse (C) senkrecht zum Mast (12).

7. Medizinische Beleuchtungsvorrichtung (1) nach Anspruch 6, **dadurch gekennzeichnet, dass** sie Anschläge (23) umfasst, die die Drehung des Gelenkarms (15) in Bezug auf den Mast (12) auf der Achse (B) parallel zum Mast (12) zwischen zwei Grenzpositionen begrenzen.

8. Medizinische Beleuchtungsvorrichtung (1) nach einem der Ansprüche 1 bis 7, **dadurch gekennzeichnet, dass** das erste Ende (13) des Mastes (12) mit dem Sockel (2) über ein Gelenk (40) verbunden ist, das eine Drehzapfenverbindung von Achse (G) aufweist, die senkrecht zur Achse (A) des Mastes (12) verläuft.

9. Medizinische Beleuchtungsvorrichtung (1) nach einem der Ansprüche 1 bis 8, **dadurch gekennzeichnet, dass** sich das erste Ende (13) des Mastes (12) in der Nähe eines Rands (13) des Sockels (2) befindet, wobei mindestens ein Gegengewicht (11) auf dem Sockel (2) gegenüber dem ersten Ende (13) des Mastes (12) montiert ist.

10. Medizinische Beleuchtungsvorrichtung (1) nach einem der Ansprüche 1 bis 9, **dadurch gekennzeichnet, dass** mindestens eines der Gelenke Verriegelungsmittel (41, 45) aufweist, die die Bewegung zwischen den entsprechenden angelenkten Elementen verhindert.

## Claims

1. A medical lighting device (1) comprising a base (2) intended to be placed on the ground, a mast (12) the first end (13) of which is connected to the base (2) and the second end (14) of which supports an arm (15), with said arm (15) comprising a first end articulated (22) on the mast (12) and a second end supporting lighting means (24), with the mast (12) being movable between a retracted position and an extended position, **characterized in that** a mounting plate (21) connects the second end (14) of the mast (12) and the first end of the arm (15), with said mounting plate (21) comprising a first portion (21a) connected to the second end (14) of the mast (12) and a second portion (21b) connected to the first end (13) of the mast (12), with both parts (21a, 21b) being able to pivot relative to each other about an axis (H) perpendicular to the mast (12).

2. A medical lighting device (1) according to claim 1, **characterized in that** the mast (12) is telescopic and comprises several segments which can slide (16) relative to each other.

3. A medical lighting device (1) according to claim 2, **characterized in that** the extension of the telescopic arm (12) is obtained by actuating a pump, for example operated manually or using a pedal (46, 47).

4. A medical lighting device (1) according to claim 2 or 3, **characterized in that** the mast (12) comprises locking means (20) making it possible to prevent the sliding of the various elements (16) of the mast (12) relative to each other.

5. A medical lighting device (1) according to one of claims 1 to 4, **characterized in that** the base (2) comprises transport wheels (4).

6. A medical lighting device according to one of claims 1 to 5, **characterized in that** the first end (13) of the arm (12) is connected to the second end of the mast through an articulation (22) comprising a pivot link having an axis (B) parallel to the mast (12) and offset from the axis (A) of the mast (12) and/or a pivot link about an axis (C) perpendicular to the mast (12).

7. A medical lighting device (1) according to claim 6, **characterized in that** it comprises stops (23) making it possible to limit the rotation of the articulated arm (15) relative to the mast (12) along the axis (B) parallel to the mast (12), between two limit positions.

8. A medical lighting device (1) according to one of claims 1 to 7, **characterized in that** the first end (13) of the mast (12) is linked to the base (2) by means of an articulation (40) comprising a pivot link having an axis (G) perpendicular to the axis (A) of the mast (12).

9. A medical lighting device (1) according to one of claims 1 to 8, **characterized in that** the first end (13) of the mast (12) is located close to an edge (13) of the base (2), with at least one counterweight (11) being mounted on the base (2), opposite the first end (13) of the mast (12).

10. A medical lighting device (1) according to one of claims 1 to 9, **characterized in that** at least one of the articulations comprises locking means (41, 45) making it possible to prevent any relative movement between the matching articulated elements.
